# EUROPEAN PATENT APPLICATION

(11) **EP 4 238 578 A1**
(43) Date of publication of application: **06.09.2023**
(21) Application number: 21886300.9
(22) Date of filing: 28.10.2021
(51) Int. Cl.: A61K 39/395, A61P 1/04, A61P 1/16, A61P 19/02, A61P 25/00, A61P 25/04, A61P 29/00, C07K 16/28, A61K 9/08, A61K 47/04, A61K 47/12, A61K 47/18, A61K 47/22, A61K 47/26, C12N 15/13, C12P 21/08

(54) **PHARMACEUTICAL COMPOSITION**

(30) Priority: 30.10.2020 JP 2020182606
(71) Applicant: Eisai R&D Management Co., Ltd, Bunkyo-ku, Tokyo 112-8088 (JP)
(72) Inventor: WATANABE, Yosuke, Tsukuba-shi, Ibaraki 300-2635 (JP)
(74) Representative: Hoffmann Eitle
(86) International application number: PCT/JP2021/039769
(87) International publication number: WO 2022/092183

(57) **Abstract**

[Problem] The purpose of the present disclosure is to provide a highly concentrated preparation of an anti-fractalkine (FKN) antibody, the preparation having viscosity that facilitates handling. [Solution] Provided is a pharmaceutical composition containing anti-FKN antibody at a concentration of 200 mg/mL. This pharmaceutical composition is characterized by containing 200 mg/mL of anti-FKN antibody and 100 to 400 mM of a basic amino acid wherein the basic amino acid comprises at least one amino acid selected from arginine, histidine, lysine, and ornithine.

## Description

### Technical Field

The present disclosure relates to a pharmaceutical composition comprising an anti-fractalkine antibody.

### Background Art

Fractalkine (also referred to as "FKN") is a membrane-bound chemokine that is expressed on the surface of vascular endothelial cells by inflammation stimulation by LPS, TNF-α, IL-1, etc. Cells that express FKN receptor CX3CR1 bind to membrane-bound FKN without intervention of selectin or integrin to cause potent cell adhesion. Moreover, secretory FKN shed from the membrane-bound FKN also shows cell migration activity against NK cells, T cells, and monocytes having CX3CR1.

Multiple mouse anti-human fractalkine (hFKN) monoclonal antibodies (clones 1F3-1, 3A5-2, 1F3, 1G1, 2B2, 3D5, 3H7, 6D1, 7F6, and 5H7-6) have been reported thus far. In particular, clone 3A5-2 has high neutralizing activity, binding affinity, and inter-species cross-reactivity against hFKN, and H3-2L4 which is the humanized antibody of the aforementioned antibody (Patent Literature 1, incorporated in its entirety by reference) is given an International Nonproprietary Name Quetmolimab.

Several formulations have thus far been disclosed in regard to a pharmaceutical composition of antibody H3-2L4 (such as Patent Literatures 2 and 3).

### Prior Art

### Patent Literatures

[Patent Literature 1] WO2011/052799
[Patent Literature 2] Japanese Published Unexamined Patent Application Publication No. 2017-193541
[Patent Literature 3] Japanese Published Unexamined Patent Application Publication No. 2018-65796

### Summary of the Invention

### Problem to be Solved by the Invention

It is thought that an anti-FKN antibody comprising H3-2L4 (herein also described as "Quetmolimab") requires administration at a high dose depending on the applicable disease. In order to increase the dosage of the anti-FKN antibody without increasing the dose of the pharmaceutical composition administered, it is necessary to raise the concentration of the anti-FKN antibody in the preparation. However, the viscosity is increased by raising the concentration of the anti-FKN antibody, and thereby the aggregability is increased, and as a result, problems arise such as development of syringe clogging, increase in slide resistance (the force required for administration increases and self-administration becomes difficult), influence on the accuracy of quantification for administration/manufacture, and reduction in packing speed for manufacture.

Accordingly, the object of the present disclosure is to provide a highly concentrated preparation of an anti-FKN antibody having viscosity that is easy to handle.

### Means for Solving the Problems

The present disclosure encompasses the following embodiments.
[1] A pharmaceutical composition comprising anti-fractalkine antibody, wherein
   said pharmaceutical composition comprises 200 mg/mL of said anti-fractalkine antibody and 100 to 400 mM of basic amino acid,
   said anti-fractalkine antibody comprises:
      a heavy chain variable region comprising the amino acid sequence shown in SEQ ID NO. 13 (QVQLVQSGAEVKKPGASVKVSCKASGYTFTNYYIHWVKQAPGQGLEWIGWIYPGDGSPKFNER FKGRTTLTADKSTNTAYMLLSSLRSEDTAVYFCATGPTDGDYFDYWGQGTTVTVSS);
      a light chain variable region comprising the amino acid sequence shown in SEQ ID NO. 14 (DIQMTQSPSSLSASVGDRVTITCRASGNIHNFLAWYQQKPGKAPKLLIYNEKTLADGVPSRFS GSGSGTDYTLTISSLQPEDFATYFCQQFWSTPYTFGGGTKVEIK); and
      the constant region of human IgG2 isotype comprising amino acid substitutions V234A and G237A in the Fc region, and said basic amino acid comprises at least one basic amino acid selected from arginine, histidine, lysine, and ornithine.
[2] The pharmaceutical composition according to [1] comprising 200 to 400 mM of basic amino acid.
[3] The pharmaceutical composition according to [1] or [2], wherein the basic amino acid comprises arginine and/or histidine.
[4] The pharmaceutical composition according to [1] or [2], wherein the basic amino acid comprises arginine.
[5] The pharmaceutical composition according to [4] comprising 250 mM of arginine.
[6] The pharmaceutical composition according to any of [1] to [5] comprising one or more buffers selected from phosphoric acid, citric acid, succinic acid, and salts thereof.
[7] The pharmaceutical composition according to [6], wherein the buffer is phosphoric acid and/or a salt thereof.
[8] The pharmaceutical composition according to any of [1] to [7] further comprising polysorbate 80.
[9] The pharmaceutical composition according to any of [1] to [8] for subcutaneous administration.
[10] The pharmaceutical composition according to any of [1] to [9], wherein the viscosity is less than 20 cP.
[11] The pharmaceutical composition according to any of [1] to [10], wherein the anti-fractalkine antibody comprises
   a heavy chain consisting of the amino acid sequence shown in SEQ ID NO. 11 
(QVQLVQSGAEVKKPGASVKVSCKASGYTFTNYYIHWVKQAPGQGLEWIGWIYPGDGSPKFNER
FKGRTTLTADKSTNTAYMLLSSLRSEDTAVYFCATGPTDGDYFDYWGQGTTVTVSSASTKGPSV
FPLAPCSRSTSESTAALGCLVKDYFPEPVTVSWNSGALTSGVHTFPAVLQSSGLYSLSSVVTVP
SSNFGTQTYTCNVDHKPSNTKVDKTVERKCCVECPPCPAPPAAAPSVFLFPPKPKDTLMISRTP
EVTCVVVDVSHEDPEVQFNWYVDGVEVHNAKTKPREEQFNSTFRVVSVLTVVHQDWLNGKEYKC
KVSNKGLPAPIEKTISKTKGQPREPQVYTLPPSREEMTKNQVSLTCLVKGFYPSDIAVEWESNG
QPENNYKTTPPMLDSDGSFFLYSKLTVDKSRWQQGNVFSCSVMHEALHNHYTQKSLSLSPGK);
 and a light chain consisting of the amino acid sequence shown in SEQ ID NO. 12 
(DIQMTQSPSSLSASVGDRVTITCRASGNIHNFLAWYQQKPGKAPKLLIYNEKTLADGVPSRFS
GSGSGTDYTLTISSLQPEDFATYFCQQFWSTPYTFGGGTKVEIKRTVAAPSVFIFPPSDEQLKS
GTASVVCLLNNFYPREAKVQWKVDNALQSGNSQESVTEQDSKDSTYSLSSTLTLSKADYEKHKV
YACEVTHQGLSSPVTKSFNRGEC).
[12] A pharmaceutical composition comprising 200 mg/mL of anti-fractalkine antibody, 250 mM of arginine, 25 mM of sodium phosphate, and 0.05 wt% of polysorbate 80, wherein said anti-fractalkine antibody comprises
   a heavy chain consisting of the amino acid sequence shown in SEQ ID NO. 11 
(QVQLVQSGAEVKKPGASVKVSCKASGYTFTNYYIHWVKQAPGQGLEWIGWIYPGDGSPKFNER
FKGRTTLTADKSTNTAYMLLSSLRSEDTAVYFCATGPTDGDYFDYWGQGTTVTVSSASTKGPSV
FPLAPCSRSTSESTAALGCLVKDYFPEPVTVSWNSGALTSGVHTFPAVLQSSGLYSLSSVVTVP
SSNFGTQTYTCNVDHKPSNTKVDKTVERKCCVECPPCPAPPAAAPSVFLFPPKPKDTLMISRTP
EVTCVVVDVSHEDPEVQFNWYVDGVEVHNAKTKPREEQFNSTFRVVSVLTVVHQDWLNGKEYKC
KVSNKGLPAPIEKTISKTKGQPREPQVYTLPPSREEMTKNQVSLTCLVKGFYPSDIAVEWESNG
 QPENNYKTTPPMLDSDGSFFLYSKLTVDKSRWQQGNVFSCSVMHEALHNHYTQKSLSLSPGK); and a light chain consisting of the amino acid sequence shown in SEQ ID NO. 12

(DIQMTQSPSSLSASVGDRVTITCRASGNIHNFLAWYQQKPGKAPKLLIYNEKTLADGVPSRFS
GSGSGTDYTLTISSLQPEDFATYFCQQFWSTPYTFGGGTKVEIKRTVAAPSVFIFPPSDEQLKS
GTASVVCLLNNFYPREAKVQWKVDNALQSGNSQESVTEQDSKDSTYSLSSTLTLSKADYEKHKV
YACEVTHQGLSSPVTKSFNRGEC).

### Effect of the Invention

According to the present disclosure, a highly concentrated preparation of an anti-FKN antibody having viscosity that is easy to handle is provided.

### Embodiments to carry out the invention

### 1. Anti-FKN antibody

In the present disclosure, when referring to an anti-FKN antibody, it refers to a humanized anti-human fractalkine antibody H3-2L4 or a functionally equivalent antibody thereof. In the present disclosure, a "functionally equivalent antibody" refers to an antibody that is equivalent to antibody H3-2L4 in at least any one or preferably all of binding affinity, neutralizing activity, and cross-reactivity against human FKN, as well as pharmacokinetics in blood.

In the present disclosure, the anti-FKN antibody comprises a heavy chain variable region comprising the amino acid sequence of SEQ ID NO. 13 (QVQLVQSGAEVKKPGASVKVSCKASGYTFTNYYIHWVKQAPGQGLEWIGWIYPGDGSPKFNER FKGRTTLTADKSTNTAYMLLSSLRSEDTAVYFCATGPTDGDYFDYWGQGTTVTVSS), and a light chain variable region comprising the amino acid sequence of SEQ ID NO. 14 (DIQMTQSPSSLSASVGDRVTITCRASGNIHNFLAWYQQKPGKAPKLLIYNEKTLADGVPSRFS GSGSGTDYTLTISSLQPEDFATYFCQQFWSTPYTFGGGTKVEIK) . In the present disclosure, an "antibody" refers to a natural or artificial immunoglobulin molecule that can recognize and bind to a specific target or antigen via at least one antigen recognition site located in its variable region.

The anti-FKN antibody according to the present disclosure is typically an antibody comprising the constant region of human IgG2 isotype. In a preferred embodiment of the present disclosure, the anti-FKN antibody according to the present disclosure comprises the constant region of human IgG2 isotype comprising V234A and/or G237A mutation in the Fc region.

In a particularly preferred embodiment of the present disclosure, the anti-FKN antibody is antibody H3-2L4 consisting of a heavy chain consisting of the amino acid sequence shown in SEQ ID NO. 11 
(QVQLVQSGAEVKKPGASVKVSCKASGYTFTNYYIHWVKQAPGQGLEWIGWIYPGDGSPKFNER
FKGRTTLTADKSTNTAYMLLSSLRSEDTAVYFCATGPTDGDYFDYWGQGTTVTVSSASTKGPSV
FPLAPCSRSTSESTAALGCLVKDYFPEPVTVSWNSGALTSGVHTFPAVLQSSGLYSLSSVVTVP
SSNFGTQTYTCNVDHKPSNTKVDKTVERKCCVECPPCPAPPAAAPSVFLFPPKPKDTLMISRTP
EVTCVVVDVSHEDPEVQFNWYVDGVEVHNAKTKPREEQFNSTFRVVSVLTVVHQDWLNGKEYKC
KVSNKGLPAPIEKTISKTKGQPREPQVYTLPPSREEMTKNQVSLTCLVKGFYPSDIAVEWESNG
QPENNYKTTPPMLDSDGSFFLYSKLTVDKSRWQQGNVFSCSVMHEALHNHYTQKSLSLSPGK),
 and a light chain consisting of the amino acid sequence shown in SEQ ID NO. 12

(DIQMTQSPSSLSASVGDRVTITCRASGNIHNFLAWYQQKPGKAPKLLIYNEKTLADGVPSRFS
GSGSGTDYTLTISSLQPEDFATYFCQQFWSTPYTFGGGTKVEIKRTVAAPSVFIFPPSDEQLKS
GTASVVCLLNNFYPREAKVQWKVDNALQSGNSQESVTEQDSKDSTYSLSSTLTLSKADYEKHKV
YACEVTHQGLSSPVTKSFNRGEC).

In another embodiment, the anti-FKN antibody is an antibody corresponding to a biosimilar when antibody H3-2L4 is the reference. A biosimilar refers to a biotherapeutic product having an equivalent/homogeneous quality, safety, and effectiveness to a preceding biotherapeutic product, and for example defined in WHO's guideline (Guidelines on evaluation of similar Biotherapeutic Products (SBPs), Annex 2, Technical Report Series No. 977, 2009) as a similar biotherapeutic product. In the aforementioned guideline, a biosimilar is defined as having the same primary structure as the reference.

An antibody where the anti-FKN antibody exemplified above was appropriately altered (such as modification of the antibody or partial substitution, addition, and deletion of the amino acid sequence of the antibody) while retaining the function of the aforementioned antibody or in order to add to or improve the function of the aforementioned antibody is also included in the antibody according to the present disclosure. More specifically, an antibody where lysine (Lys) located at the carboxy terminal (C-terminal) of the heavy chain is deleted by an artificial method such as genetic modification in order to decrease the ununiformity of antibodies produced by antibody-producing cells is also included in the scope of the present disclosure. Moreover, the anti-FKN antibodies contained in the pharmaceutical composition according to the present disclosure do not necessarily need to have complete uniformity, and for example, those where lysine (Lys) located at the carboxy terminal (C-terminal) of the heavy chain is deleted and not deleted may be coexist, as long as the function intended by the pharmaceutical composition according to the present disclosure is maintained.

The anti-FKN antibody may be modified as desired. The anti-FKN antibody may be a modification that changes (a) the three-dimensional structure of the amino acid sequence in the modification region such as the sheet or helix conformation; (b) the molecular charge or hydrophobicity state at the target site; or (c) the effect of modification on maintaining the side chain volume, or may be a modification in which these changes are not clearly observed.

The modification of the anti-FKN antibody may be achieved by e.g. substitution, deletion, addition, and the like of the configuring amino acid residues.

In the present specification, an amino acid is employed in its broadest meaning, and includes not only natural amino acids, e.g. serine (Ser), asparagine (Asn), valine (Val), leucine (Leu), isoleucine (Ile), alanine (Ala), tyrosine (Tyr), glycine (Gly), lysine (Lys), arginine (Arg), histidine (His), aspartic acid (Asp), glutamic acid (Glu), glutamine (Gin), threonine (Thr), cysteine (Cys), methionine (Met), phenylalanine (Phe), tryptophan (Trp), and proline (Pro), but also non-natural amino acids such as amino acid variants and derivatives. Those skilled in the art will naturally recognize that in consideration of this broad definition, the amino acids herein include e.g. L-amino acids; D-amino acids; chemically modified amino acids such as amino acid variants and amino acid derivatives; amino acids that will not be components of protein in the body such as norleucine, β-alanine, and ornithine; as well as chemically synthesized compounds having amino acid properties well-known to those skilled in the art, and the like. Examples of non-natural amino acids include α-methylamino acids (such as α-methylalanine), D-amino acids (such as D-aspartic acid and D-glutamic acid), histidine-like amino acids (such as 2-amino-histidine, β-hydroxy-histidine, homohistidine, α-fluoromethyl-histidine, and a-methyl-histidine), amino acids having excess methylenes on the side chain ("homo" amino acids), and amino acids where the carboxylate functional group amino acid in the side chain is substituted with a sulfonate group (such as cysteic acid), and the like.

For example, naturally-occurring amino acid residues may be classified into the following groups based on general side chain properties:
(1) hydrophobic: Met, Ala, Val, Leu, Ile;
(2) neutral hydrophilic: Cys, Ser, Thr;
(3) acidic: Asp, Glu;
(4) basic: Asn, Gln, His, Lys, Arg;
(5) residues that influence chain orientation: Gly, Pro; and
(6) aromatic: Trp, Tyr, Phe.

A nonconservative substitution of an amino acid sequence that configures an anti-FKN antibody may be carried out by exchanging an amino acid belonging to one of these groups with an amino acid belonging to another group. A more conservative substitution may be carried out by exchanging an amino acid belonging to one of these groups with an amino acid in the same group. Similarly, deletion or substitution of an amino acid sequence may be appropriately carried out.

### 2. Pharmaceutical composition and preparation

The present disclosure relates to a highly concentrated preparation of an anti-FKN antibody. The pharmaceutical composition according to the present disclosure is based on the knowledge that by adding a particular basic amino acid, the anti-FKN antibody can be prepared at a high concentration while having low viscosity and superior operativity.

In the present disclosure, the amount of specific anti-FKN antibody may vary depending on the administration route and the administration interval of the pharmaceutical composition according to the present disclosure, and the present disclosure particularly relates to a pharmaceutical composition comprising 200 mg/mL of anti-FKN antibody.

A basic amino acid referred to herein may comprise any of L-isomer or D-isomer. In one embodiment of the present disclosure, the basic amino acid is an L-amino acid.

Basic amino acids that can be used in the present disclosure can include arginine, histidine, lysine, and ornithine. One or more types of said basic amino acid can be used in combination. In one embodiment of the present disclosure, a basic amino acid comprising arginine, histidine, lysine, or ornithine, or a combination of two or more types thereof is used. In one embodiment of the present disclosure, arginine, histidine, lysine, or ornithine, or a combination of two or more types thereof is used. In one embodiment of the present disclosure, arginine, histidine, or lysine, or a combination of two or more types thereof is used.

In another embodiment of the present disclosure, a combination of arginine, histidine, or arginine and histidine is used as the basic amino acid.

In yet another embodiment of the present disclosure, arginine is used as the basic amino acid.

In the present disclosure, the concentration of use of the basic amino acid will vary depending on the type of the basic amino acid selected, the administration route, and the like, an can be selected from a range of 25 mM to 500 mM, such as a range of 50 mM to 400 mM, 100 mM to 400 mM, 200 mM to 400 mM, 250 mM to 400 mM, 300 mM to 400 mM, 50 mM to 300 mM, 100 mM to 300 mM, 200 mM to 300 mM, 250 mM to 300 mM, 50 mM to 250 mM, 100 mM to 250 mM, 200 mM to 250 mM, 50 mM to 200 mM, or 100 mM to 200 mM.

In one embodiment, the preparation according to the present disclosure contains arginine at a concentration of about 50 mM or 50 mM, about 100 mM or 100 mM, about 200 mM or 200 mM, about 250 mM or 250 mM, about 300 mM or 300 mM, about 400 mM or 400 mM.

In one embodiment, the preparation according to the present disclosure contains histidine at a concentration of about 50 mM or 50 mM, about 100 mM or 100 mM, about 200 mM or 200 mM, about 250 mM or 250 mM, about 300 mM or 300 mM, about 400 mM or 400 mM.

In one embodiment, the pharmaceutical composition according to the present disclosure contains a combination of two or more amino acids selected from the group consisting of arginine, histidine, lysine, and ornithine at a concentration of a total of about 50 mM or 50 mM, a total of about 100 mM or 100 mM, a total of about 200 mM or 200 mM, a total of about 250 mM or 250 mM, a total of about 300 mM or 300 mM, or a total of about 400 mM or 400 mM. In another embodiment, the aforementioned combination of two or more amino acids is selected from the group consisting of arginine, histidine, and lysine. In one embodiment, the pharmaceutical composition according to the present disclosure contains the combination of histidine and arginine at a concentration of a total of about 50 mM or 50 mM, a total of about 100 mM or 100 mM, a total of about 200 mM or 200 mM, a total of about 250 mM or 250 mM, a total of about 300 mM or 300 mM, or a total of about 400 mM or 400 mM. In another embodiment, the pharmaceutical composition according to the present disclosure contains a combination of histidine at a concentration of about 50 mM or 50 mM and arginine at a concentration of about 250 mM or 250 mM, a combination of histidine at a concentration of about 100 mM or 100 mM and arginine at a concentration of about 200 mM or 200 mM, a combination of histidine at a concentration of about 150 mM or 150 mM and arginine at a concentration of about 150 mM or 150 mM, or a combination of histidine at a concentration of about 250 mM or 50 mM and arginine at a concentration of about 250 mM or 50 mM.

In the present disclosure, a pharmaceutical composition comprising an anti-FKN antibody and a basic amino acid comprises a buffer. The buffer can include a buffer comprising phosphoric acid, citric acid, succinic acid, L-histidine, acetic acid, gluconic acid, Tris, glycine, or a salt thereof, or a combination thereof, and is specifically an acid or base and/or a salt thereof. When both an acid or base and a salt are added, the salt may be the salt of the same or a different acid or base. Moreover, pH adjusters routinely used by those skilled in the art such as potassium carbonate, sodium hydrogen carbonate, hydrochloric acid, and sodium hydroxide may be employed in order to adjust the pH of the pharmaceutical composition. In one embodiment, the pharmaceutical composition in the present disclosure has pH in the range of about 4.5 to about 6.5. In one embodiment, the pH is in the pH range of 5.0 to 6.25, in the range of pH 5.25 to 6.00, or in the range of pH 5.5 to 5.8. In a particular embodiment of the present disclosure, the pharmaceutical composition according to the present disclosure has pH 5.8 or about 5.8.

The concentration of the buffer can be about 1 mM to about 600 mM depending on the type of the buffer used and the desired isotonicity.

In one embodiment, the buffer is phosphoric acid and/or a salt thereof (such as sodium phosphate) at a concentration of about 5 mM to about 40 mM, or about 10 mM to about 30 mM, or about 15 mM to about 25 mM. In another embodiment, the buffer is sodium phosphate at a concentration of about 25 mM or 25 mM.

In one embodiment, the buffer is citric acid and/or a salt thereof (such as sodium citrate) at a concentration of about 5 mM to about 40 mM, or about 10 mM to about 30 mM, or about 15 mM to about 25 mM. In another embodiment, the buffer is citric acid at a concentration of about 25 mM or 25 mM.

In one embodiment, the buffer is succinic acid and/or a salt thereof (such as succinic acid sodium) at a concentration of about 5 mM to about 40 mM, or about 10 mM to about 30 mM, or about 15 mM to about 25 mM. In another embodiment, the buffer is succinic acid at a concentration of about 25 mM or 25 mM.

In one embodiment, the buffer is L-histidine and/or a salt thereof at a concentration of about 5 mM to about 40 mM, or about 10 mM to about 30 mM, or about 15 mM to about 25 mM. In another embodiment, the buffer is L-histidine at a concentration of about 25 mM or 25 mM.

The pharmaceutical composition according to the present disclosure can optionally be prepared together with stabilizers, surfactants, preservatives, and the like routinely used in the aforementioned technical field.

An example of a stabilizer used in the present disclosure are carbohydrates or saccharides or sugars recognized by the authority to be an appropriate additive in a pharmaceutical preparation, such as sucrose. The concentration of the stabilizer can be 15 to 250 mM, or 150 to 250 mM, or 200 mM. The pharmaceutical composition according to the present disclosure may comprise a secondary stabilizer.

Appropriate examples of pharmaceutically acceptable surfactants used in the present disclosure include, but are not limited to, polyoxyethylene sorbitan fatty acid ester (Tween), polyethylene polypropylene glycol, polyoxyethylene stearate, and polyoxyethylene alkyl ether, such as polyoxyethylene monolauryl ether, alkyl phenyl polyoxyethylene ether (Triton-X), polyoxyethylene/polyoxypropylene copolymer (Poloxamer, Pluronic), and dodecyl sodium sulfate (SDS). The most appropriate polyoxyethylene sorbitan fatty acid esters are polysorbate 20 (Tween 20) and polysorbate 80 (Tween 80). The concentration of the surfactant is 0.01 to 0.1% (w/v), or 0.01 to 0.08% (w/v), or 0.025 to 0.075% (w/v), such as 0.05% (w/v).

In one embodiment of the present disclosure, polysorbate 80 is used as the surfactant.

Preservatives used in the present disclosure include, but are not limited to, paraben, benzyl alcohol, sodium benzoate, phenol, benzalkonium chloride, thimerosal, chlorobutanol, benzoic acid, sodium hydrogen sulfite, sodium propionate, and any other combination or mixture, and the like.

In addition to the above additives, representative additives and processes are well-known in the art, and reference can be made to e.g. Introduction to Pharmaceutical Dosage Forms, 1985, Ansel, H.C., Lea and Febiger, Philadelphia, Pa.; Remington's Pharmaceutical Sciences, 1995, Mack Publ. Co., Easton, Pa. The above literatures are herein incorporated in their entirety by reference.

The pharmaceutical composition according to the present disclosure containing the above ingredients can have a viscosity of less than 25 centipoise (cP), preferably less than 20 cP. Note that in the present disclosure, viscosity (cP) refers to the value at 25°C. The viscosity (cP) of the pharmaceutical composition according to the present disclosure can be determined with the aforementioned well-known method in the art such as capillary viscometry or rotation viscometry employing an instrument or device appropriate for the aforementioned method. In one embodiment, it is capillary viscometry, and the specific method, the device etc. used herein are described in the Examples.

Moreover, the pharmaceutical composition according to the present disclosure containing the above ingredients may be a pharmaceutical composition with low aggregability of anti-FKN antibodies.

Accordingly, the present disclosure encompasses a method of preparing a pharmaceutical composition containing an anti-FKN antibody, comprising adjusting a pharmaceutical composition comprising 200 mg/mL of anti-FKN antibody to have a viscosity of less than 25 centipoise (cP) or less than 20 cP. Moreover, the present disclosure encompasses a method of lowering the viscosity of a pharmaceutical composition comprising 200 mg/mL of anti-FKN antibody to less than 25 centipoise (cP) or less than 20 cP.

The pharmaceutical composition according to the present disclosure can be administered by a parenteral route such as subcutaneous, intravenous, or intramuscular. In one embodiment, the pharmaceutical composition according to the present disclosure is a pharmaceutical composition for subcutaneous administration.

The pharmaceutical composition according to the present disclosure may be provided in a state filled in a vial or an administration container (syringe) in advance.

The pharmaceutical composition according to the present disclosure can be used for treating diseases associated with FKN, such as inflammatory diseases including rheumatoid arthritis, ulcerative colitis, Crohn's disease, autoimmune hepatitis, multiple sclerosis, and neuropathic pain.

### Specific preferred embodiments will be described below.

In a preferred embodiment, the pharmaceutical composition according to the present disclosure comprises 200 mg/mL of anti-FKN antibody, 50 mM of arginine, 25 mM of sodium phosphate, and 0.05 wt% of polysorbate 80, wherein said anti-fractalkine antibody comprises
a heavy chain consisting of the amino acid sequence shown in SEQ ID NO. 11 
(QVQLVQSGAEVKKPGASVKVSCKASGYTFTNYYIHWVKQAPGQGLEWIGWIYPGDGSPKFNER
FKGRTTLTADKSTNTAYMLLSSLRSEDTAVYFCATGPTDGDYFDYWGQGTTVTVSSASTKGPSV
FPLAPCSRSTSESTAALGCLVKDYFPEPVTVSWNSGALTSGVHTFPAVLQSSGLYSLSSVVTVP
SSNFGTQTYTCNVDHKPSNTKVDKTVERKCCVECPPCPAPPAAAPSVFLFPPKPKDTLMISRTP
EVTCVVVDVSHEDPEVQFNWYVDGVEVHNAKTKPREEQFNSTFRVVSVLTVVHQDWLNGKEYKC
KVSNKGLPAPIEKTISKTKGQPREPQVYTLPPSREEMTKNQVSLTCLVKGFYPSDIAVEWESNG
QPENNYKTTPPMLDSDGSFFLYSKLTVDKSRWQQGNVFSCSVMHEALHNHYTQKSLSLSPGK);
 and a light chain consisting of the amino acid sequence shown in SEQ ID NO. 12

(DIQMTQSPSSLSASVGDRVTITCRASGNIHNFLAWYQQKPGKAPKLLIYNEKTLADGVPSRFS
GSGSGTDYTLTISSLQPEDFATYFCQQFWSTPYTFGGGTKVEIKRTVAAPSVFIFPPSDEQLKS
GTASVVCLLNNFYPREAKVQWKVDNALQSGNSQESVTEQDSKDSTYSLSSTLTLSKADYEKHKV
YACEVTHQGLSSPVTKSFNRGEC).

In another preferred embodiment, the pharmaceutical composition according to the present disclosure comprises 200 mg/mL of anti-FKN antibody, 100 mM of arginine, 25 mM of sodium phosphate, and 0.05 wt% of polysorbate 80, wherein said anti-fractalkine antibody comprises
a heavy chain consisting of the amino acid sequence shown in SEQ ID NO. 11 
(QVQLVQSGAEVKKPGASVKVSCKASGYTFTNYYIHWVKQAPGQGLEWIGWIYPGDGSPKFNER
FKGRTTLTADKSTNTAYMLLSSLRSEDTAVYFCATGPTDGDYFDYWGQGTTVTVSSASTKGPSV
FPLAPCSRSTSESTAALGCLVKDYFPEPVTVSWNSGALTSGVHTFPAVLQSSGLYSLSSVVTVP
SSNFGTQTYTCNVDHKPSNTKVDKTVERKCCVECPPCPAPPAAAPSVFLFPPKPKDTLMISRTP
EVTCVVVDVSHEDPEVQFNWYVDGVEVHNAKTKPREEQFNSTFRVVSVLTVVHQDWLNGKEYKC
KVSNKGLPAPIEKTISKTKGQPREPQVYTLPPSREEMTKNQVSLTCLVKGFYPSDIAVEWESNG
QPENNYKTTPPMLDSDGSFFLYSKLTVDKSRWQQGNVFSCSVMHEALHNHYTQKSLSLSPGK);
 and a light chain consisting of the amino acid sequence shown in SEQ ID NO. 12

(DIQMTQSPSSLSASVGDRVTITCRASGNIHNFLAWYQQKPGKAPKLLIYNEKTLADGVPSRFS
GSGSGTDYTLTISSLQPEDFATYFCQQFWSTPYTFGGGTKVEIKRTVAAPSVFIFPPSDEQLKS
GTASVVCLLNNFYPREAKVQWKVDNALQSGNSQESVTEQDSKDSTYSLSSTLTLSKADYEKHKV
YACEVTHQGLSSPVTKSFNRGEC).

In another preferred embodiment, the pharmaceutical composition according to the present disclosure comprises 200 mg/mL of anti-FKN antibody, 300 mM of arginine, 25 mM of sodium phosphate, and 0.05 wt% of polysorbate 80, wherein said anti-fractalkine antibody comprises
a heavy chain consisting of the amino acid sequence shown in SEQ ID NO. 11 
(QVQLVQSGAEVKKPGASVKVSCKASGYTFTNYYIHWVKQAPGQGLEWIGWIYPGDGSPKFNER
FKGRTTLTADKSTNTAYMLLSSLRSEDTAVYFCATGPTDGDYFDYWGQGTTVTVSSASTKGPSV
FPLAPCSRSTSESTAALGCLVKDYFPEPVTVSWNSGALTSGVHTFPAVLQSSGLYSLSSVVTVP
SSNFGTQTYTCNVDHKPSNTKVDKTVERKCCVECPPCPAPPAAAPSVFLFPPKPKDTLMISRTP
EVTCVVVDVSHEDPEVQFNWYVDGVEVHNAKTKPREEQFNSTFRVVSVLTVVHQDWLNGKEYKC
KVSNKGLPAPIEKTISKTKGQPREPQVYTLPPSREEMTKNQVSLTCLVKGFYPSDIAVEWESNG
QPENNYKTTPPMLDSDGSFFLYSKLTVDKSRWQQGNVFSCSVMHEALHNHYTQKSLSLSPGK);
 and a light chain consisting of the amino acid sequence shown in SEQ ID NO. 12

(DIQMTQSPSSLSASVGDRVTITCRASGNIHNFLAWYQQKPGKAPKLLIYNEKTLADGVPSRFS
GSGSGTDYTLTISSLQPEDFATYFCQQFWSTPYTFGGGTKVEIKRTVAAPSVFIFPPSDEQLKS
GTASVVCLLNNFYPREAKVQWKVDNALQSGNSQESVTEQDSKDSTYSLSSTLTLSKADYEKHKV
YACEVTHQGLSSPVTKSFNRGEC).

In another preferred embodiment, the pharmaceutical composition according to the present disclosure comprises 200 mg/mL of anti-FKN antibody, 400 mM of arginine, 25 mM of sodium phosphate, and 0.05 wt% of polysorbate 80, wherein said anti-fractalkine antibody comprises
a heavy chain consisting of the amino acid sequence shown in SEQ ID NO. 11 
(QVQLVQSGAEVKKPGASVKVSCKASGYTFTNYYIHWVKQAPGQGLEWIGWIYPGDGSPKFNER
FKGRTTLTADKSTNTAYMLLSSLRSEDTAVYFCATGPTDGDYFDYWGQGTTVTVSSASTKGPSV
FPLAPCSRSTSESTAALGCLVKDYFPEPVTVSWNSGALTSGVHTFPAVLQSSGLYSLSSVVTVP
SSNFGTQTYTCNVDHKPSNTKVDKTVERKCCVECPPCPAPPAAAPSVFLFPPKPKDTLMISRTP
EVTCVVVDVSHEDPEVQFNWYVDGVEVHNAKTKPREEQFNSTFRVVSVLTVVHQDWLNGKEYKC
KVSNKGLPAPIEKTISKTKGQPREPQVYTLPPSREEMTKNQVSLTCLVKGFYPSDIAVEWESNG
QPENNYKTTPPMLDSDGSFFLYSKLTVDKSRWQQGNVFSCSVMHEALHNHYTQKSLSLSPGK);
 and a light chain consisting of the amino acid sequence shown in SEQ ID NO. 12

(DIQMTQSPSSLSASVGDRVTITCRASGNIHNFLAWYQQKPGKAPKLLIYNEKTLADGVPSRFS
GSGSGTDYTLTISSLQPEDFATYFCQQFWSTPYTFGGGTKVEIKRTVAAPSVFIFPPSDEQLKS
GTASVVCLLNNFYPREAKVQWKVDNALQSGNSQESVTEQDSKDSTYSLSSTLTLSKADYEKHKV
YACEVTHQGLSSPVTKSFNRGEC).

In another preferred embodiment, the pharmaceutical composition according to the present disclosure comprises 200 mg/mL of anti-FKN antibody, 250 mM of lysine, 25 mM of sodium phosphate, and 0.05 wt% of polysorbate 80, wherein said anti-fractalkine antibody comprises
a heavy chain consisting of the amino acid sequence shown in SEQ ID NO. 11 
(QVQLVQSGAEVKKPGASVKVSCKASGYTFTNYYIHWVKQAPGQGLEWIGWIYPGDGSPKFNER
FKGRTTLTADKSTNTAYMLLSSLRSEDTAVYFCATGPTDGDYFDYWGQGTTVTVSSASTKGPSV
FPLAPCSRSTSESTAALGCLVKDYFPEPVTVSWNSGALTSGVHTFPAVLQSSGLYSLSSVVTVP
SSNFGTQTYTCNVDHKPSNTKVDKTVERKCCVECPPCPAPPAAAPSVFLFPPKPKDTLMISRTP
EVTCVVVDVSHEDPEVQFNWYVDGVEVHNAKTKPREEQFNSTFRVVSVLTVVHQDWLNGKEYKC
KVSNKGLPAPIEKTISKTKGQPREPQVYTLPPSREEMTKNQVSLTCLVKGFYPSDIAVEWESNG
QPENNYKTTPPMLDSDGSFFLYSKLTVDKSRWQQGNVFSCSVMHEALHNHYTQKSLSLSPGK);
 and a light chain consisting of the amino acid sequence shown in SEQ ID NO. 12

(DIQMTQSPSSLSASVGDRVTITCRASGNIHNFLAWYQQKPGKAPKLLIYNEKTLADGVPSRFS
GSGSGTDYTLTISSLQPEDFATYFCQQFWSTPYTFGGGTKVEIKRTVAAPSVFIFPPSDEQLKS
GTASVVCLLNNFYPREAKVQWKVDNALQSGNSQESVTEQDSKDSTYSLSSTLTLSKADYEKHKV
YACEVTHQGLSSPVTKSFNRGEC).

In another preferred embodiment, the pharmaceutical composition according to the present disclosure comprises 200 mg/mL of anti-FKN antibody, 250 mM of histidine, 25 mM of sodium phosphate, and 0.05 wt% of polysorbate 80, wherein said anti-fractalkine antibody comprises
a heavy chain consisting of the amino acid sequence shown in SEQ ID NO. 11 
(QVQLVQSGAEVKKPGASVKVSCKASGYTFTNYYIHWVKQAPGQGLEWIGWIYPGDGSPKFNER
FKGRTTLTADKSTNTAYMLLSSLRSEDTAVYFCATGPTDGDYFDYWGQGTTVTVSSASTKGPSV
FPLAPCSRSTSESTAALGCLVKDYFPEPVTVSWNSGALTSGVHTFPAVLQSSGLYSLSSVVTVP
SSNFGTQTYTCNVDHKPSNTKVDKTVERKCCVECPPCPAPPAAAPSVFLFPPKPKDTLMISRTP
EVTCVVVDVSHEDPEVQFNWYVDGVEVHNAKTKPREEQFNSTFRVVSVLTVVHQDWLNGKEYKC
KVSNKGLPAPIEKTISKTKGQPREPQVYTLPPSREEMTKNQVSLTCLVKGFYPSDIAVEWESNG
QPENNYKTTPPMLDSDGSFFLYSKLTVDKSRWQQGNVFSCSVMHEALHNHYTQKSLSLSPGK);
 and a light chain consisting of the amino acid sequence shown in SEQ ID NO. 12

(DIQMTQSPSSLSASVGDRVTITCRASGNIHNFLAWYQQKPGKAPKLLIYNEKTLADGVPSRFS
GSGSGTDYTLTISSLQPEDFATYFCQQFWSTPYTFGGGTKVEIKRTVAAPSVFIFPPSDEQLKS
GTASVVCLLNNFYPREAKVQWKVDNALQSGNSQESVTEQDSKDSTYSLSSTLTLSKADYEKHKV
YACEVTHQGLSSPVTKSFNRGEC).

In another preferred embodiment, the pharmaceutical composition according to the present disclosure comprises 200 mg/mL of anti-FKN antibody, 250 mM of arginine, 25 mM of sodium phosphate, and 0.05 wt% of polysorbate 80, wherein said anti-fractalkine antibody comprises
a heavy chain consisting of the amino acid sequence shown in SEQ ID NO. 11 
(QVQLVQSGAEVKKPGASVKVSCKASGYTFTNYYIHWVKQAPGQGLEWIGWIYPGDGSPKFNER
FKGRTTLTADKSTNTAYMLLSSLRSEDTAVYFCATGPTDGDYFDYWGQGTTVTVSSASTKGPSV
FPLAPCSRSTSESTAALGCLVKDYFPEPVTVSWNSGALTSGVHTFPAVLQSSGLYSLSSVVTVP
SSNFGTQTYTCNVDHKPSNTKVDKTVERKCCVECPPCPAPPAAAPSVFLFPPKPKDTLMISRTP
EVTCVVVDVSHEDPEVQFNWYVDGVEVHNAKTKPREEQFNSTFRVVSVLTVVHQDWLNGKEYKC
KVSNKGLPAPIEKTISKTKGQPREPQVYTLPPSREEMTKNQVSLTCLVKGFYPSDIAVEWESNG
QPENNYKTTPPMLDSDGSFFLYSKLTVDKSRWQQGNVFSCSVMHEALHNHYTQKSLSLSPGK);
 and a light chain consisting of the amino acid sequence shown in SEQ ID NO. 12

(DIQMTQSPSSLSASVGDRVTITCRASGNIHNFLAWYQQKPGKAPKLLIYNEKTLADGVPSRFS
GSGSGTDYTLTISSLQPEDFATYFCQQFWSTPYTFGGGTKVEIKRTVAAPSVFIFPPSDEQLKS
GTASVVCLLNNFYPREAKVQWKVDNALQSGNSQESVTEQDSKDSTYSLSSTLTLSKADYEKHKV
YACEVTHQGLSSPVTKSFNRGEC).

Those skilled in the art will recognize that as long as it is not technically contradicting, any one or more of any and all aspects described herein may be appropriately combined to carry out the present disclosure. Further, those skilled in the art will recognize that as long as it is not technically contradicting, it will be preferred that any and all preferred or advantageous aspects described herein are appropriately combined to carry out the present disclosure.

All of the disclosures of the literatures cited herein should be deemed as clearly cited herein by reference, and those skilled in the art can cite and recognize the related disclosed contents in these literatures as a part of the present specification according to the context herein without departing from the spirit and scope of the present disclosure.

The literatures cited herein are provided solely for the purpose of disclosing related technology preceding the filing date of the present application, and are not to be construed as an admission by the present inventors that the present invention does not hold the right to precede said disclosures due to prior inventions or for any other reason. All descriptions in these literatures are based on the information available to the present applicants, and do not configure in any way an admission that the contents of these descriptions are accurate.

The terms used herein are employed for describing particular embodiments, and do not intend to limit the invention.

The terms "comprise, include" employed herein, unless the content clearly indicates to be understood otherwise, intend the presence of the described items (such as components, steps, elements, or numbers), and do not exclude the presence of other items (such as components, steps, elements, and numbers). The term "consist of" encompasses the aspects described with the terms "consist of" and/or "consist essentially of."

Unless otherwise defined, all terms used herein (including technical and scientific terms) have the same meaning as that broadly recognized by those skilled in the art of the technology to which the present disclosure belongs. The terms used herein, unless explicitly defined otherwise, should be construed as having meanings consistent with the meanings herein and in related technical fields, and shall not be construed as having idealized or excessively formal meanings.

Terms such as first and second are employed to express various elements, and it should be recognized that these elements are not to be limited by these terms per se. These terms are employed solely for the purpose of discriminating one element from another, and for example, it is possible to describe a first element as a second element, and similarly, to describe a second element as a first element without departing from the scope of the present disclosure.

In the specification and claims herein, the numeric values employed for indicating a component content or a numeric value range and the like, unless explicitly indicated, are to be understood as being modified by the term "about." For example, "10 µg," unless explicitly indicated, is recognized as meaning "about 10 µg,'' and it is natural that those skilled in the art can reasonably recognize the extent thereof according to technical common sense and the meaning of the present specification.

Unless clearly indicated to mean otherwise in context, when used in the specification and claims herein, it should be recognized that each aspect represented in singular form may also be a plural form as long as it is not technically contradicting, and vice versa.

The present disclosure will now be described in further detail with reference to Examples. However, the present disclosure can be embodied by various aspects, and shall not be construed as being limited to the Examples described herein. Those skilled in the art of related technical fields can carry out the present disclosure without changing the spirit and scope of the present disclosure with various modifications, additions, deletions, substitution, and the like.

### Examples

### Example 1: Preparation of humanized anti-human fractalkine antibody

In the following administration test to humans, humanized anti-human fractalkine antibody H3-2L4 was employed. The production of H3-2L4 including humanization was carried out as described in WO2011/052799. H3-2L4 used in Example 2 and on were prepared with the methods described in 1-1. and 1-2. below.

### 1-1. Expression vector

The production of the expression vector for humanized anti-human fractalkine antibody (H3-2L4) was carried out as follows.

First, a signal sequence (SEQ ID NO. 3) was added to the N-terminal of the amino acid sequence of the heavy chain variable region (H3-2) of humanized anti-human fractalkine antibody (H3-2L4) (SEQ ID NO. 1), and the amino acid sequence of the constant region of human IgG2 having two mutations (V234A and G237A) inserted (SEQ ID NO. 4) was added to the C-terminal (SEQ ID NO. 5) . Next, a signal sequence (SEQ ID NO. 6) was added to the N-terminal of the amino acid sequence of the light chain variable region (L4) of humanized anti-human fractalkine antibody (H3-2L4) (SEQ ID NO. 2), and the amino acid sequence of the constant region of human Igκ (SEQ ID NO. 7) was added to the C-terminal (SEQ ID NO. 8). These amino acid sequences (SEQ ID NOs. 5 and 8) were converted into a gene sequence optimal for expression in CHO cells, and a sequence having the recognition sequence of the restriction enzyme HindIII and the Kosak sequence added to the 5'-terminal of the gene sequence, and a stop codon and the recognition sequence of the restriction enzyme EcoRI added to the 3'-terminal, respectively, was totally synthesized (SEQ ID NOs. 9 and 10).

Note that sequences specified by SEQ ID NOs. 1 to 10 were each as follows.
Amino acid sequence of heavy chain variable region (H3-2) (SEQ ID NO. 1) 
QVQLVQSGAEVKKPGASVKVSCKASGYTFTNYYIHWVKQAPGQGLEWIGWIYPGDGSPKFNERF
KGRTTLTADKSTNTAYMLLSSLRSEDTAVYFCATGPTDGDYFDYWGQGTTVTVSS
Amino acid sequence of light chain variable region (L4) (SEQ ID NO. 2) 
DIQMTQSPSSLSASVGDRVTITCRASGNIHNFLAWYQQKPGKAPKLLIYNEKTLADGVPSRFSG
SGSGTDYTLTISSLQPEDFATYFCQQFWSTPYTFGGGTKVEIK
Signal sequence (SEQ ID NO. 3)
   MEWSWVFLFFLSVTTGVHS
Amino acid sequence of constant region of human IgG2 having two mutations (V234A and G237A) inserted (SEQ ID NO. 4)

ASTKGPSVFPLAPCSRSTSESTAALGCLVKDYFPEPVTVSWNSGALTSGVHTFPAVLQSSGLYS
LSSVVTVPSSNFGTQTYTCNVDHKPSNTKVDKTVERKCCVECPPCPAPPAAAPSVFLFPPKPKD
TLMISRTPEVTCVVVDVSHEDPEVQFNWYVDGVEVHNAKTKPREEQFNSTFRVVSVLTVVHQDW
LNGKEYKCKVSNKGLPAPIEKTISKTKGQPREPQVYTLPPSREEMTKNQVSLTCLVKGFYPSDI

AVEWESNGQPENNYKTTPPMLDSDGSFFLYSKLTVDKSRWQQGNVFSCSVMHEALHNHYTQKSL
SLSPGK

MEWSWVFLFFLSVTTGVHSQVQLVQSGAEVKKPGASVKVSCKASGYTFTNYYIHWVKQAPGQGL
EWIGWIYPGDGSPKFNERFKGRTTLTADKSTNTAYMLLSSLRSEDTAVYFCATGPTDGDYFDYW
GQGTTVTVSSASTKGPSVFPLAPCSRSTSESTAALGCLVKDYFPEPVTVSWNSGALTSGVHTFP
AVLQSSGLYSLSSVVTVPSSNFGTQTYTCNVDHKPSNTKVDKTVERKCCVECPPCPAPPAAAPS
VFLFPPKPKDTLMISRTPEVTCVVVDVSHEDPEVQFNWYVDGVEVHNAKTKPREEQFNSTFRVV
SVLTVVHQDWLNGKEYKCKVSNKGLPAPIEKTISKTKGQPREPQVYTLPPSREEMTKNQVSLTC
LVKGFYPSDIAVEWESNGQPENNYKTTPPMLDSDGSFFLYSKLTVDKSRWQQGNVFSCSVMHEA
LHNHYTQKSLSLSPGK

(SEQ ID NO. 5) Signal sequence (SEQ ID NO. 6)
   MSVPTQVLGLLLLWLTDARC
Amino acid sequence of constant region of human Igκ (SEQ ID NO. 7)
(SEQ ID NO. 8) 
MSVPTQVLGLLLLWLTDARCDIQMTQSPSSLSASVGDRVTITCRASGNIHNFLAWYQQKPGKAP
KLLIYNEKTLADGVPSRFSGSGSGTDYTLTISSLQPEDFATYFCQQFWSTPYTFGGGTKVEIKR
TVAAPSVFIFPPSDEQLKSGTASVVCLLNNFYPREAKVQWKVDNALQSGNSQESVTEQDSKDST
YSLSSTLTLSKADYEKHKVYACEVTHQGLSSPVTKSFNRGEC
(SEQ ID NO. 9) 
AAGCTTGCCGCCACCATGGAATGGTCCTGGGTGTTCCTGTTCTTCCTGTCCGTGACCACCGGCG
TGCACTCCCAGGTGCAGCTGGTGCAGTCTGGCGCCGAAGTGAAGAAACCTGGCGCCTCCGTGAA
GGTGTCCTGCAAGGCCTCCGGCTACACCTTCACCAACTACTACATCCACTGGGTGAAACAGGCC
CCAGGACAGGGCCTGGAATGGATCGGCTGGATCTACCCCGGCGACGGCTCCCCCAAGTTCAACG
AGCGGTTCAAGGGCCGGACCACCCTGACCGCCGACAAGTCCACCAACACCGCCTACATGCTGCT
GTCCTCCCTGCGGAGCGAGGATACCGCCGTGTACTTCTGCGCCACCGGCCCTACCGACGGCGAC
TACTTCGACTACTGGGGCCAGGGCACCACCGTGACCGTGTCCTCTGCCTCCACCAAGGGCCCCT
CCGTGTTCCCTCTGGCCCCTTGCTCCCGGTCCACCTCTGAGTCTACCGCCGCTCTGGGCTGCCT
GGTCAAAGACTACTTCCCCGAGCCTGTGACAGTGTCCTGGAACTCTGGCGCCCTGACCTCTGGA
 
GTGCATACCTTCCCTGCCGTGCTGCAGTCATCCGGCCTGTACTCCCTGTCCTCCGTGGTGACAG
TGCCCTCCTCCAACTTCGGCACCCAGACCTACACCTGTAACGTGGACCACAAGCCCTCCAACAC
CAAGGTGGACAAGACCGTGGAACGGAAGTGCTGCGTGGAATGCCCCCCCTGTCCTGCCCCTCCT
GCCGCCGCTCCTTCCGTGTTTCTGTTCCCCCCAAAGCCCAAGGACACCCTGATGATCTCCCGGA
CCCCCGAAGTGACCTGCGTGGTGGTGGACGTGTCCCACGAGGACCCCGAGGTGCAGTTCAATTG
GTACGTGGACGGCGTGGAAGTGCACAACGCCAAGACCAAGCCCAGAGAGGAACAGTTCAACTCC
ACCTTCCGGGTGGTGTCCGTGCTGACCGTGGTGCACCAGGACTGGCTGAACGGCAAAGAGTACA
AGTGCAAGGTCTCCAACAAGGGCCTGCCTGCCCCCATCGAAAAGACCATCAGCAAGACCAAGGG
CCAGCCCCGCGAGCCCCAGGTGTACACACTGCCCCCCAGCCGGGAAGAGATGACCAAGAACCAG
GTGTCCCTGACCTGTCTGGTGAAAGGCTTCTACCCCTCCGATATCGCCGTGGAATGGGAGTCCA
ACGGACAGCCCGAGAACAACTACAAGACCACCCCCCCCATGCTGGACTCCGACGGCTCATTCTT
CCTGTACTCCAAGCTGACAGTGGACAAGTCCCGGTGGCAGCAGGGCAACGTGTTCTCCTGCAGC
GTGATGCACGAGGCCCTGCACAACCACTACACCCAGAAGTCCCTGAGCCTGAGCCCCGGCAAGT
GATGAATTC
(SEQ ID NO. 10)

AAGCTTGCCGCCACCATGTCCGTGCCCACCCAGGTGCTGGGCCTGCTGCTGCTGTGGCTGACCG
ACGCCAGATGCGACATCCAGATGACCCAGTCCCCCTCCAGCCTGTCCGCCTCTGTGGGCGACAG
AGTGACCATCACCTGTCGGGCCTCCGGCAACATCCACAACTTTCTGGCCTGGTATCAGCAGAAG
CCCGGCAAGGCCCCCAAGCTGCTGATCTACAACGAAAAGACCCTGGCCGACGGCGTGCCCTCCA
GATTCTCCGGCTCTGGCTCCGGCACCGACTACACCCTGACCATCTCCAGCCTGCAGCCCGAGGA
CTTCGCCACCTACTTTTGCCAGCAGTTCTGGTCCACCCCCTACACCTTCGGCGGAGGCACCAAG
GTGGAAATCAAGCGGACCGTGGCCGCTCCCTCCGTGTTCATCTTCCCACCCTCCGACGAGCAGC
TGAAGTCCGGCACCGCCTCCGTGGTGTGCCTGCTGAACAACTTCTACCCCCGCGAGGCCAAGGT
GCAGTGGAAGGTGGACAACGCCCTGCAGTCCGGCAACTCCCAGGAATCCGTCACCGAGCAGGAC
TCCAAGGACAGCACCTACTCCCTGTCCTCCACCCTGACCCTGTCCAAGGCCGACTACGAGAAGC
ACAAGGTGTACGCCTGCGAAGTGACCCACCAGGGCCTGTCCAGCCCCGTGACCAAGTCCTTCAA
CCGGGGCGAGTGCTGATGAATTC

The gene sequence encoding totally synthesized heavy chain was cleaved out with restriction enzymes HindIII and EcoRI, and inserted into HindIII and EcoRI sites of a pEE6.4 vector (Lonza). The gene sequence encoding totally synthesized light chain was cleaved out with restriction enzymes HindIII and EcoRI, and inserted into HindIII and EcoRI sites of a pEE12.4 vector (Lonza). Each vector was cleaved with restriction enzymes NotI and PvuI, and vector fragments comprising the heavy chain and the light chain were ligated with each other to construct the expression vector.

### 1-2. Construction of cell strain expressing antibody H3-2L4 and acquisition of antibody H3-2L4

The expression vector produced was transferred into CHOK1SV cells (Lonza) conditioned in CD-CHO/6 mM L-glutamine medium by electroporation method. After the gene transfer, selection was carried out in CD-CHO/50 uM MSX medium under 37°C/10% CO₂ environment, and cells expressing the antibody of interest were acquired. The cells were then cloned to produce a cell bank. The cell bank produced was resuscitated/cultured, the culture supernatant was purified by chromatography, and the antibody of interest H3-2L4 was acquired.

Antibody H3-2L4 acquired according to the above had heavy chain and light chain having amino acid sequences shown in SEQ ID NOs. 11 and 12, respectively.
Full length heavy chain of H3-2L4 (SEQ ID NO. 11) 
QVQLVQSGAEVKKPGASVKVSCKASGYTFTNYYIHWVKQAPGQGLEWIGWIYPGDGSPKFNERF
KGRTTLTADKSTNTAYMLLSSLRSEDTAVYFCATGPTDGDYFDYWGQGTTVTVSSASTKGPSVF
PLAPCSRSTSESTAALGCLVKDYFPEPVTVSWNSGALTSGVHTFPAVLQSSGLYSLSSVVTVPS
SNFGTQTYTCNVDHKPSNTKVDKTVERKCCVECPPCPAPPAAAPSVFLFPPKPKDTLMISRTPE
VTCVVVDVSHEDPEVQFNWYVDGVEVHNAKTKPREEQFNSTFRVVSVLTVVHQDWLNGKEYKCK
VSNKGLPAPIEKTISKTKGQPREPQVYTLPPSREEMTKNQVSLTCLVKGFYPSDIAVEWESNGQ
PENNYKTTPPMLDSDGSFFLYSKLTVDKSRWQQGNVFSCSVMHEALHNHYTQKSLSLSPGK
Full length light chain of H3-2L4 (SEQ ID NO. 12)

DIQMTQSPSSLSASVGDRVTITCRASGNIHNFLAWYQQKPGKAPKLLIYNEKTLADGVPSRFSG
SGSGTDYTLTISSLQPEDFATYFCQQFWSTPYTFGGGTKVEIKRTVAAPSVFIFPPSDEQLKSG
TASVVCLLNNFYPREAKVQWKVDNALQSGNSQESVTEQDSKDSTYSLSSTLTLSKADYEKHKVY
ACEVTHQGLSSPVTKSFNRGEC

Moreover, the amino acid sequences of the heavy chain variable region and the light chain variable region of antibody H3-2L4 were each as follows.
Heavy chain variable region of H3-2L4 (SEQ ID NO. 13) 
QVQLVQSGAEVKKPGASVKVSCKASGYTFTNYYIHWVKQAPGQGLEWIGWIYPGDGSPKFNERF
KGRTTLTADKSTNTAYMLLSSLRSEDTAVYFCATGPTDGDYFDYWGQGTTVTVSS
Light chain variable region of H3-2L4 (SEQ ID NO. 14)

DIQMTQSPSSLSASVGDRVTITCRASGNIHNFLAWYQQKPGKAPKLLIYNEKTLADGVPSRFSG
SGSGTDYTLTISSLQPEDFATYFCQQFWSTPYTFGGGTKVEIK

Moreover, the amino acid sequences of CDR-H1 to CDR-H3 and CDR-L1 to CDR-L3 of antibody H3-2L4 were as follows:
CDR-H1 of H3-2L4 (SEQ ID NO. 15)
   NYYIH
CDR-H2 of H3-2L4 (SEQ ID NO. 16)
   WIYPGDGSPKFNERFKG
CDR-H3 of H3-2L4 (SEQ ID NO. 17)
   GPTDGDYFDY
CDR-L1 of H3-2L4 (SEQ ID NO. 18)
   RASGNIHNFLA
CDR-L2 of H3-2L4 (SEQ ID NO. 19)
   NEKTLAD
CDR-L3 of H3-2L4 (SEQ ID NO. 20) QQFWSTPYT

### Example 2 Investigation of formulation that lowers viscosity

In the presence of 25 mM sodium phosphate, citric acid, succinic acid, or L-histidine, 200 mg/mL preparations of antibody H3-2L4 were adjusted to within a pH range of 5.0 to 6.5 with various additives, and the viscosity was measured (Table 1 to Table 4). Aqueous solutions of HCl and NaOH were used for the adjustment of pH.

**[Table 1]**

| Formulation list for measuring viscosity 1 (influence of various additives) | | | | | | |
|---|---|---|---|---|---|---|
| Entr y | Active ingredien t | Buffer | Additive | Surfactant | pH | Viscosit y (cP) |
| 1 | H3-2L4 200 mg/mL | 25 mM sodium phosphat e | No additive | 0.05 wt% polysorbat e 80 | 5. 8 | >50 |
| 2 | | | 50 mM arginine | | | 22.6 |
| 3 | | | 100 mM arginine | | | 17.7 |
| 4 | | | 200 mM arginine | | | 14.0 |
| 5 | | | 300 mM arginine | | | 11.5 |
| 6 | | | 400 mM arginine | | | 10.3 |
| 7 | | | 250 mM lysine | | | 19.4 |
| 8 | | | 250 mM ornithine | | | 21.5 |
| 9 | | | 250 mM histidine | | | 11.9 |
| 10 | | | 250 mM NaCl | | | 41.8 |
| 11 | | | 250 mM glycine | | | 43.0 |
| 12 | | | 250 mM proline | | | 37.2 |
| 13 | | | 250 mM methionin e | | | 40.3 |
| 14 | | | 250 mM glutamic acid | | | 39.2 |
| 15 | | | 250 mM citrullin e | | | 35.1 |

**[Table 2]**

| Formulation list for measuring viscosity 2 (influence of L-arginine/L-histidine) | | | | | | |
|---|---|---|---|---|---|---|
| Entr y | Active ingredien t | Buffer | Additive | Surfactant | pH | Viscosit y (cP) |
| 1 | H3-2L4 200 mg/mL | 25 mM sodium phosphat e | No additive | 0.05 wt% polysorbat e 80 | 5. 8 | >50 |
| 2 | | | 50 mM arginine | | | 22.6 |
| 3 | | | 100 mM arginine | | | 17.7 |
| 4 | | | 200 mM arginine | | | 14.0 |
| 5 | | | 300 mM arginine | | | 11.5 |
| 6 | | | 400 mM arginine | | | 10.3 |
| 7 | | | 50 mM histidin e | | | 21.4 |
| 8 | | | 100 mM histidin e | | | 14.2 |
| 9 | | | 200 mM histidin e | | | 10.9 |
| 10 | | | 300 mM histidin e | | | 10.5 |
| 11 | | | 400 mM histidin e | | | 9.7 |
| 12 | | 50 mM histidine + 250 mM arginine | | NA | | 10.8 |
| 13 | | 100 mM histidine + 200 mM arginine | | | | 10.9 |
| 14 | | 150 mM histidine + 150 mM arginine | | | | 10.2 |
| 15 | | 250 mM histidine + 50 mM arginine | | | | 10.6 |
| 16 | | 25 mM sodium phosphat e | 250 mM arginine | 0.05 wt% polysorbat e 80 | | 11.6 |

**[Table 3]**

| Formulation list for measuring viscosity 3 (influence of buffer) | | | | | | |
|---|---|---|---|---|---|---|
| Entr y | Active ingredien t | Buffer | Additive | Surfactant | pH | Viscosit y (cP) |
| 1 | H3-2L4 200 mg/mL | 25 mM sodium phosphat e | 250 mM L-arginin e | 0.05 wt% polysorbat e 80 | 5. 8 | 11.6 |
| 2 | | 25 mM histidin e | | NA | | 11.1 |
| 3 | | 25 mM citric acid | | | | 11.7 |
| 4 | | 25 mM succinic acid | | | | 11.5 |

**[Table 4]**

| Formulation list for measuring viscosity 4 (influence of pH of preparation) | | | | | |
|---|---|---|---|---|---|
| Entry | Active ingredient | Buffer | Additive | Surfactant | pH |
| 1 | H3-2L4 200 mg/mL | 25 mM sodium phosphate | 250 mM L-arginine | 0.05 wt% polysorbate 80 | 5.0 |
| 2 | | | | | 5.6 |
| 3 | | | | | 5.8 |
| 4 | | | | | 6.0 |
| 5 | | | | | 6.5 |

The viscosity was measured with capillary method. Specifically, measurement was made by capillary method employing PA800 plus (Beckman Coulter) equipped with a photodiode array detector. A base fused-silica capillary with an inner diameter of 50 µm was employed. The capillary was filled for 5 minutes at 80 psi with viscosity standard solution (5, 10, and 50 cP) or antibody H3-2L4 preparation sample. Subsequently, a small amount of 0.02 wt% riboflavin was loaded onto the capillary and employed as the pigment to monitor the movement of the sample solution. A constant pressure of 70 psi was applied to the measurement device having the sample vial mounted at the entrance of the capillary. The movement of the pigment to the detection window was detected at 445 nm. The standard curve was obtained according to the transfer time of riboflavin in the viscosity standard solution (5, 10, and 50 cP). Viscosity was determined from the migration time of riboflavin peak moving through the capillary filled with the sample. Note that all measurements were carried out at 25°C.

### Results

Viscosity was extremely reduced by addition of basic amino acids (L-arginine, L-histidine, lysine, and ornithine) (Table 1, Nos. 2 to 9). Among the preparations employing basic amino acids, formulations employing L-histidine and L-arginine especially had large viscosity lowering effects (Table 1). In particular, large viscosity lowering effects were observed in preparations comprising L-histidine at 200 mM or more or L-arginine at 200 mM or more. (Table 2, Nos. 1 to 11 and 16)

On the other hand, an effect by a combination of L-arginine and L-histidine was not observed (Table 2, Nos. 12 to 15). Moreover, the buffers did not influence the viscosity of the solution (Table 3) .

In regard to pH, the viscosity at pH 5.6, 5.8, and 6.0 (Table 4) were 11.9 cP, 11.6 cP, and 11.1 cP, respectively.

## Claims

1. A pharmaceutical composition comprising anti-fractalkine antibody, wherein
said pharmaceutical composition comprises 200 mg/mL of said anti-fractalkine antibody and 100 to 400 mM of basic amino acid,
said anti-fractalkine antibody comprises:
a heavy chain variable region comprising the amino acid sequence shown in SEQ ID NO. 13 (QVQLVQSGAEVKKPGASVKVSCKASGYTFTNYYIHWVKQAPGQGLEWIGWIYPGDGSPKFNER FKGRTTLTADKSTNTAYMLLSSLRSEDTAVYFCATGPTDGDYFDYWGQGTTVTVSS);
a light chain variable region comprising the amino acid sequence shown in SEQ ID NO. 14 (DIQMTQSPSSLSASVGDRVTITCRASGNIHNFLAWYQQKPGKAPKLLIYNEKTLADGVPSRFS GSGSGTDYTLTISSLQPEDFATYFCQQFWSTPYTFGGGTKVEIK); and
the constant region of human IgG2 isotype comprising amino acid substitutions V234A and G237A in the Fc region, and
said basic amino acid comprises at least one basic amino acid selected from arginine, histidine, lysine, and ornithine.

2. The pharmaceutical composition according to claim 1 comprising 200 to 400 mM of basic amino acid.

3. The pharmaceutical composition according to claim 1 or 2, wherein the basic amino acid comprises arginine and/or histidine.

4. The pharmaceutical composition according to claim 1 or 2, wherein the basic amino acid comprises arginine.

5. The pharmaceutical composition according to claim 4 comprising 250 mM of arginine.

6. The pharmaceutical composition according to any one of claims 1 to 5 comprising one or more buffers selected from phosphoric acid, citric acid, succinic acid, and salts thereof.

7. The pharmaceutical composition according to claim 6, wherein the buffer is phosphoric acid and/or a salt thereof.

8. The pharmaceutical composition according to any one of claims 1 to 7 further comprising polysorbate 80.

9. The pharmaceutical composition according to any one of claims 1 to 8 for subcutaneous administration.

10. The pharmaceutical composition according to any one of claims 1 to 9, wherein the viscosity is less than 20 cP.

11. The pharmaceutical composition according to any one of claims 1 to 10, wherein the anti-fractalkine antibody comprises
a heavy chain consisting of the amino acid sequence shown in SEQ ID NO. 11 
(QVQLVQSGAEVKKPGASVKVSCKASGYTFTNYYIHWVKQAPGQGLEWIGWIYPGDGSPKFNER
FKGRTTLTADKSTNTAYMLLSSLRSEDTAVYFCATGPTDGDYFDYWGQGTTVTVSSASTKGPSV
FPLAPCSRSTSESTAALGCLVKDYFPEPVTVSWNSGALTSGVHTFPAVLQSSGLYSLSSVVTVP
SSNFGTQTYTCNVDHKPSNTKVDKTVERKCCVECPPCPAPPAAAPSVFLFPPKPKDTLMISRTP
EVTCVVVDVSHEDPEVQFNWYVDGVEVHNAKTKPREEQFNSTFRVVSVLTVVHQDWLNGKEYKC
KVSNKGLPAPIEKTISKTKGQPREPQVYTLPPSREEMTKNQVSLTCLVKGFYPSDIAVEWESNG
QPENNYKTTPPMLDSDGSFFLYSKLTVDKSRWQQGNVFSCSVMHEALHNHYTQKSLSLSPGK);
 and a light chain consisting of the amino acid sequence shown in SEQ ID NO. 12

(DIQMTQSPSSLSASVGDRVTITCRASGNIHNFLAWYQQKPGKAPKLLIYNEKTLADGVPSRFS
GSGSGTDYTLTISSLQPEDFATYFCQQFWSTPYTFGGGTKVEIKRTVAAPSVFIFPPSDEQLKS
GTASVVCLLNNFYPREAKVQWKVDNALQSGNSQESVTEQDSKDSTYSLSSTLTLSKADYEKHKV
YACEVTHQGLSSPVTKSFNRGEC).

12. A pharmaceutical composition comprising 200 mg/mL of anti-fractalkine antibody, 250 mM of arginine, 25 mM of sodium phosphate, and 0.05 wt% of polysorbate 80, wherein said anti-fractalkine antibody comprises
a heavy chain consisting of the amino acid sequence shown in SEQ ID NO. 11 
(QVQLVQSGAEVKKPGASVKVSCKASGYTFTNYYIHWVKQAPGQGLEWIGWIYPGDGSPKFNER
FKGRTTLTADKSTNTAYMLLSSLRSEDTAVYFCATGPTDGDYFDYWGQGTTVTVSSASTKGPSV
FPLAPCSRSTSESTAALGCLVKDYFPEPVTVSWNSGALTSGVHTFPAVLQSSGLYSLSSVVTVP
SSNFGTQTYTCNVDHKPSNTKVDKTVERKCCVECPPCPAPPAAAPSVFLFPPKPKDTLMISRTP
EVTCVVVDVSHEDPEVQFNWYVDGVEVHNAKTKPREEQFNSTFRVVSVLTVVHQDWLNGKEYKC
KVSNKGLPAPIEKTISKTKGQPREPQVYTLPPSREEMTKNQVSLTCLVKGFYPSDIAVEWESNG
QPENNYKTTPPMLDSDGSFFLYSKLTVDKSRWQQGNVFSCSVMHEALHNHYTQKSLSLSPGK);
 and a light chain consisting of the amino acid sequence shown in SEQ ID NO. 12
